(19) Europäisches Patentamt
European Patent Office
Office européen des brevets

(11) EP 1 614 689 A1

(12) EUROPEAN PATENT APPLICATION
published in accordance with Art. 158(3) EPC

(43) Date of publication:
**11.01.2006 Bulletin 2006/02**

(21) Application number: **04727399.0**

(22) Date of filing: **14.04.2004**

(51) Int Cl.:
***C07D 491/044*** *(1985.01)* *A61K 31/553* *(2000.01)*
*A61P 37/08* *(2000.01)*

(86) International application number:
**PCT/JP2004/005304**

(87) International publication number:
**WO 2004/092178 (28.10.2004 Gazette 2004/44)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **15.04.2003 JP 2003109892**

(71) Applicant: **Fujiyakuhin Co., Ltd.**
**Saitama-shi, Saitama 331-8508 (JP)**

(72) Inventors:
- **UDA, Junichiro,**
**FUJIYAKUHIN CO., LTD. RES. LAB. 1**
**Saitama-shi,**
**Saitama 331-0047 (JP)**
- **SASAKI, Tomomitsu,**
**FUJIYAKUHIN CO., LTD RES LAB. 1**
**Saitama-shi,**
**Saitama 331-0047 (JP)**
- **SATO, Takahiro,**
**FUJIYAKUHIN CO., LTD. RES LAB. 1**
**Saitama-shi,**
**Saitama 331-0047 (JP)**
- **INOUE, Tsutomu,**
**FUJIYAKUHIN CO., LTD. RES. LAB. 1**
**Saitama-shi,**
**Saitama 331-0047 (JP)**

(74) Representative: **Hartz, Nikolai et al**
**Wächtershauser & Hartz**
**Weinstrasse 8**
**80333 München (DE)**

# (54) BENZOXEPINO-11-PIPERIDYLIDENE COMPOUNDS AND PROCESS FOR PRODUCTION THEREOF

(57) Provided are a process for producing an acid addition salt of a 3-[4-(8-fluoro-5,11-dihydrobenz[b]oxepino[4,3-b]pyridin-11-ylidene)piperidino]propionic acid alkyl ester by reacting a 3-[4-(8-fluoro-5,11-dihydrobenz[b]oxepino[4,3-b]pyridin-11-ylidene)piperidino]propionic acid alkyl ester with an acid, and a process for producing 3-[4-(8-fluoro-5,11-dihydrobenz[b]oxepino[4,3-b]pyridin-11-ylidene)piperidino]propionic acid using the acid addition salt as an intermediate.

By using as an intermediate an acid addition salt of a 3-[4-(8-fluoro-5,11-dihydrobenz[b]oxepino[4,3-b]pyridin-11-ylidene)piperidino]propionic acid alkyl ester to produce 3-[4-(8-fluoro-5,11-dihydrobenz[b]oxepino[4,3-b]pyridin-11-ylidene)piperidino]propionic acid, the metals used in the synthetic reaction steps and the organic compounds mainly by-produced during production are readily separated from a reaction liquid by a simple procedure, and the by-products are sufficiently removed without using a purification step by chromatography, thereby enabling mass production and enhancing production efficiency.

EP 1 614 689 A1

## Description

### Technical Field

The present invention relates to novel acid addition salts of 3-[4-(8-fluoro-5,11-dihydrobenz[b]oxepino[4,3-b]pyridin-11-ylidene)piperidino]propionic acid alkyl esters which are an intermediate for synthesizing 3-[4-(8-fluoro-5,11-dihydrobenz[b]oxepino[4,3-b]pyridin-11-ylidene)piperidino]propionic acid which is useful as an antiallergic agent of amphoteric type, as well as a process for production thereof and utilization thereof.

### Background Art

It is known that 3-[4-(8-fluoro-5,11-dihydrobenz[b]oxepino[4,3-b]pyridin-11-ylidene)piperidino]propionic acid is useful as an antiallergic agent of amphoteric type (for example, see JP 6-192263 A and Journal of Medicinal Chemistry, Vol.38, No.3, pages 496-507). It is also known that, as an improved process for producing this compound, 8-fluoro-11-oxo-5,11-dihydrobenz[b]oxepino[4,3-b]pyridine is reacted with 3-(4-oxo-piperidin-1-yl)-propionic acid ethyl ester in the presence of a low-valency titanium to give 3-[4-(8-fluoro-5,11-dihydrobenz[b]oxepino[4,3-b]pyridin-11-ylidene)piperidino]propionic acid ethyl ester and then the resulting compound is hydrolyzed, whereby the process steps are largely reduced, the reaction yield and the overall yield are largely improved, and the production efficiency is remarkably enhanced (see JP 2000-338574 A).

In the process described in JP 2000-338574 A which is an improved process, 8-fluoro-11-oxo-5,11-dihydrobenz[b]oxepino[4,3-b]pyridine is reacted with 3-(4-oxo-piperidin-1-yl)-propionic acid ethyl ester to give 3-[4-(8-fluoro-5,11-dihydrobenz[b]oxepino[4,3-b]pyridin-11-ylidene)piperidino]propionic acid ethyl ester, water and a base are added thereto, and the product is extracted with an organic solvent and then hydrolyzed to obtain an aimed compound. According to this process, however, a muddy insoluble matter is formed during the extraction. It was revealed that the insoluble matter is difficult to remove by filtration and, particularly in an industrial scale production, separation of the insoluble matter by filtration is very difficult. In addition, it was revealed that a column purification step is necessary to remove the metals used in the process and the organic impurities which are mainly by-produced during production and thus the process is industrially disadvantageous.

For the above-mentioned reasons, it has been desired to develop an effective process for producing 3-[4-(8-fluoro-5,11-dihydrobenz[b]oxepino[4,3-b]pyridin-11-ylidene)piperidino]propionic acid or an acid addition salt thereof having such a purity that it can be used as a medicament on an industrial scale.

Disclosure of the Invention

An object of the present invention is to provide a process for producing 3-[4-(8-fluoro-5,11-dihydrobenz[b]oxepino[4,3-b]pyridin-11-ylidene)piperidino]propionic acid or an acid addition salt thereof having a high purity by effectively removing impurities and by-products which are produced or remain in the process, an intermediate for producing thereof, and a process for producing the intermediate.

The present inventors have recognized the importance of the process intermediate to minimize the time and loss of compounds in the process for production of 3-[4-(8-fluoro-5,11-dihydrobenz[b]oxepino[4,3-b]pyridin-11-ylidene)piperidino]propionic acid or an acid addition salt thereof and to improve the production efficiency thereof and have intensively researched particularly on the purification step after the reaction. As a result, it has been found a process which goes through a novel acid addition salt of a 3-[4-(8-fluoro-5,11-dihydrobenz[b]oxepino[4,3-b]pyridin-11-ylidene)piperidino]propionic acid alkyl ester is excellent for separating the metals used in a synthetic reaction step and the by-products mainly accompanied by the production from the reaction liquid to complete the invention.

The present invention provides an acid addition salt of a 3-[4-(8-fluoro-5,11-dihydrobenz[b]oxepino[4,3-b]pyridin-11-ylidene)piperidino]propionic acid alkyl ester.

The present invention provides a process for producing an acid addition salt of a 3-[4-(8-fluoro-5,11-dihydrobenz[b]oxepino[4,3-b]pyridin-11-ylidene)piperidino]propionic acid alkyl ester which comprises reacting a 3-[4-(8-fluoro-5,11-dihydrobenz[b]oxepino[4,3-b]pyridin-11-ylidene)piperidino]propionic acid alkyl ester with an acid.

Further, the present invention provides a process for producing 3-[4-(8-fluoro-5,11-dihydrobenz[b]oxepino[4,3-b]pyridin-11-ylidene)piperidino]propionic acid or an acid addition salt thereof which comprises hydrolyzing an acid addition salt of a 3-[4-(8-fluoro-5,11-dihydrobenz[b]oxepino[4,3-b]pyridin-11-ylidene)piperidino]propionic acid alkyl ester.

### Best Mode for Carrying out the Invention

The alkyl group in the acid addition salt of a 3-[4-(8-fluoro-5,11-dihydrobenz[b]oxepino[4,3-b]pyridin-11-ylidene)piperidino]propionic acid alkyl ester (hereinafter, sometimes referred to as benzoxepino-11-piperidylidene compound), which is an ester residue of the benzoxepino-11-piperidylidene compound, is preferably a straight or branched C1 to C5 alkyl group, particularly ethyl group.

The acid addition salt of a 3-[4-(8-fluoro-5,11-dihydrobenz[b]oxepino[4,3-b]pyridin-11-ylidene)piperidino]propionic acid alkyl ester includes inorganic acid salts such as hydrochlorides, hydrobromides, phosphates and sulfates, and organic acid salts such as methanesulfonates, p-toluenesulfonates and oxalates. 3-[4-(8-Fluoro-5,11-dihydrobenz[b]oxepino[4,3-b]pyridin-11-ylidene)piperidino]propionic acid ethyl ester hydrochloride is most preferable as the benzoxepino-11-piperidylidene compound.

The reaction scheme of the process for production of 3-[4-(8-fluoro-5,11-dihydrobenz[b]oxepino[4,3-b]pyridin-11-ylidene)piperidino]propionic acid or an acid addition salt which goes through a benzoxepino-11-piperidylidene compound, a novel intermediate of the present invention, is as follows:

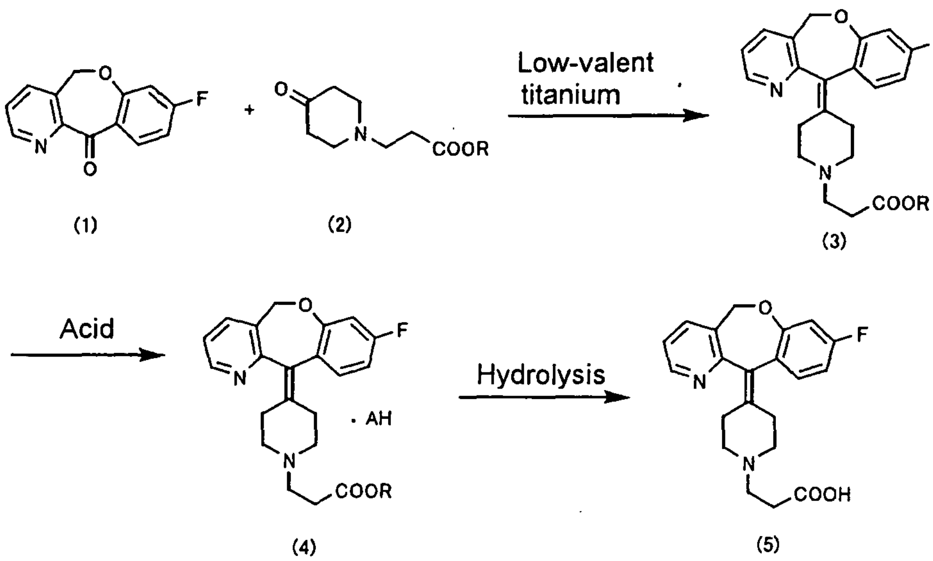

(1) (2) (3) (4) (5)

wherein, R denotes an alkyl and AH denotes an acid.

Namely, 8-fluoro-11-oxo-5,11-dihydrobenz[b]oxepino[4,3-b]pyridin (1) is reacted with a 3-(4-oxo-piperidin-1-yl)-propionic acid alkyl ester (2) in the presence of a low-valent titanium to give a 3-[4-(8-fluoro-5,11-dihydrobenz[b]oxepino[4,3-b]pyridin-11-ylidene)piperidino]propionic acid alkyl ester (3), the resulting ester (3) is reacted with an acid to give the benzoxepino-11-piperidylidene compound (4) according to the present invention, and then the compound (4) is hydrolyzed to give 3-[4-(8-fluoro-5,11-dihydrobenz[b]oxepino[4,3-b]pyridin-11-ylidene)piperidino]propionic acid (5) or an acid addition salt thereof.

The reaction between compound (1) and compound (2) is effected by adding a mixed solution of compound (1) and compound (2) to a liquid mixture containing a low-valency titanium. The low-valent titanium used herein means a titanium having a valency of lower than 3 and may be generated in the reaction system by using a reducing agent and a halogenated trivalent or tetravalent titanium. Examples of the halogenated titanium include titanium chlorides such as titanium tetrachloride and titanium trichloride; and titanium bromides. Examples of the reducing agent include zinc, a zinc-copper alloy, magnesium, lithium and lithium aluminum hydride. As the low-valent titanium, it is preferred, for example, to use those produced by reacting zinc or a zinc-copper alloy with titanium tetrachloride or titanium trichloride in the reaction system. The reaction between compound (1) and compound (2) is preferably carried out, in view of the yield of compound (3), by adding compound (1) and compound (2) to a heated mixture which is obtained by reacting a halogenated trivalent or tetravalent titanium with a reducing agent such as, for example, zinc or a zinc-copper alloy, preferably a heated mixture at a temperature in a range of from the temperature which is lower than the boiling point of the mixture by 10°C(boiling point minus 10°C) to the boiling point of the mixture.

Compound (3) is preferably used after the reaction liquid for synthesis is stirred with air bubbles in the presence of an organic base and then insoluble matters are separated from the reaction liquid. Examples of the organic base include amines, nitrogen-containing heterocyclic compounds and the like. The amines include mono (C1 to C6)alkylamines, di (C1 to C6)alkylamines, and tri(C1 to C6)alkylamines. The mono-, di- or tri-alkylamine is preferably triethylamine, tripropylamine, diisopropylethylamine or the like and is particularly preferably triethylamine.

The time for stirring with air bubbles depends on reaction scale, but is preferably 0.5 to 5 hours, particularly 1 to 1.5 hours when air flow per kg of compound (1) is 25 to 200 L/min, particularly 30 to 70 L/min. Stirring with air bubbles

prevents the reaction liquid from becoming viscous and makes the subsequent filtration procedure after addition of water smooth.

Separation of compound (3) from the reaction liquid is preferably effected by extraction with a water-organic solvent mixture. Examples of the organic solvent include low fatty acid esters such as ethyl acetate, propyl acetate, isopropyl acetate and butyl acetate. Ethyl acetate is preferred as the organic solvent.

The mixing ratio of water to the organic solvent, a ratio of water:organic solvent by volume, is preferably 1:2 to 2:1, and more preferably about 1:1.

Reaction of compound (3) with an acid gives the benzoxepino-11-piperidylidene compound of the present invention.

Examples of the acid include inorganic acids such as hydrogen chloride, hydrogen bromide, phosphoric acid and sulfuric acid; and organic acids such as methanesulfonic acid, p-toluenesulfonic acid and oxalic acid. Preferred acids include hydrogen chloride, hydrogen bromide, methanesulfonic acid and p-toluenesulfonic acid.

The molar ratio of compound (3) to the acid is preferably in a range of from 1:1.5 to 1.5:1, more preferably in a range of from 1:1.1 to 1.1:1.

The reaction is preferably conducted with heating, more preferably with heating under reflux. Heating temperature is preferably from 30°C to a reflux temperature of the solvent used, particularly from 70°C to the reflux temperature. The time of the heating or heating under reflux varies depending on the reaction scale but is preferably 5 minutes to 1 hour, more preferably 5 to 20 minutes.

When compound (3) is mixed with the acid, the acid, particularly hydrogen chloride or hydrogen bromide, is preferably dissolved in an organic solvent. Examples of the organic solvent include lower alcohols such as ethanol, 1-propanol, 2-propanol and n-butanol; and lower fatty acid esters such as ethyl acetate, propyl acetate, isopropyl acetate and butyl acetate. Ethanol, 2-propanol and ethyl acetate are preferred as the organic solvent.

The benzoxepino-11-piperidylidene compound (4) is particularly preferably prepared by mixing compound (3) dissolved in an organic solvent and the acid dissolved in an organic acid, heating the mixture under reflux, cooling the mixture, and filtering the mixture.

If purification of benzoxepino-11-piperidylidene compound (4) is insufficient, compound (4) is again incorporated with an organic solvent, heated preferably under reflux and then cooled to enhance purification efficiency. The organic solvent used may be any as long as it can dissolve benzoxepino-11-piperidylidene compound (4) during heating, and is preferably ethanol, 2-propanol or ethyl acetate.

By such simple procedure, it is possible to remove the metals used in the previous step and organic by-products from benzoxepino-11-piperidylidene compound (4). Since metals and organic by-products are sufficiently removed by simple operation without necessity of chromatography used in the production process described in JP 2000-338574 A, mass production is enabled and an actual production efficiency in a factory is enhanced.

By hydrolyzing benzoxepino-11-piperidylidene compound (4), 3-[4-(8-Fluoro-5,11-dihydrobenz[b]oxepino[4,3-b]pyridin-11-ylidene)piperidino]propionic acid (hereinafter, sometimes referred to as merely piperidylidene propionic acid)(5) or an acid addition salt thereof is produced.

The hydrolysis is preferably conducted using an acid or a base. As the acid can be used hydrochloric acid, hydrobromic acid, sulfuric acid or phosphoric acid, and as the base can be used sodium hydroxide, potassium hydroxide or potassium carbonate.

The amount of the acid or the base used for the hydrolysis is preferably not less than 2 moles, more preferably 2 to 4 moles per mole of benzoxepino-11-piperidylidene compound (4).

Examples of the acid addition salt of piperidylidene propionic acid (5) include hydrochloride, hydrobromide, tartrate, methanesulfonate, and citrate.

## Examples

Degree of purification of an organic compound was measured by using high performance liquid chromatography (HPLC, product of Nippon Bunko) using acetonitrile, methanol or the like as a solvent and that of residual metals was measured by high-frequency plasma emission spectrometry (for example, OPTIM A-3300DV supplied by Parkin Elmer Inc., U.S.A.).

Example 1

3-[4-(8-Fluoro-5,11-dihydrobenz[b]oxepino[4,3-b]pyridin-11-ylidene)piperidino]propionic acid ethyl ester

To a suspension of zinc (17.8 g) in anhydrous tetrahydrofuran (180 mL) was added dropwise titanium tetrachloride (9.65 mL) under ice-cooling in an argon atmosphere. After the reaction mixture was stirred under reflux for 2 hours, to the boiling mixture was promptly added a solution of 8-fluoro-11-oxo-5,11-dihydrobenz[b]oxepino[4,3-b]pyridin

(10.0 g) and 3-(4-oxopiperidin-1-yl)-propionic acid ethyl ester (8.7 g) in anhydrous tetrahydrofuran (135 mL) under reflux. After heating under reflux for 30 minutes, the reaction mixture was cooled to room temperature, incorporated with triethylamine (56.5 mL) and ethyl acetate (350 mL), and stirred with bubbles of air stream at 1 L/min under stirring at room temperature for 60 minutes. Precipitated insoluble matters were filtered through celite and washed twice with ethyl acetate (75 mL). The filtrate and the washing solution were combined and concentrated under reduced pressure, and to the residue was added ethyl acetate (350 mL) and water (350 mL), stirred at room temperature for 10 minutes. Precipitated insoluble matters were filtered through celite and washed twice with ethyl acetate (30 mL). An organic layer was separated from the combined mixture of the filtrate and the washing solution, an aqueous layer was extracted with ethyl acetate (100 mL), and the organic layer was combined therewith, washed with brine (75 mL), and then dried over anhydrous magnesium sulfate. After concentrating under reduced pressure, 8.65 g of the aimed product (85.8%, HPLC) was obtained as a brown viscous oil.
HPLC retention time: 5.9 minutes [column: Crestpack C18 T-5, 200mm; solvent: acetonitrile-0.1% aqueous phosphoric acid solution (containing 5 mmol/L of sodium 1-heptanesulfonate) (35:65), detection: UV (258 nm), flow rate; 1.0 mL/min]]

Example 2

3-[4-(8-Fluoro-5,11-dihydrobenz[b]oxepino[4,3-b]pyridin-11-ylidene)piperidino]propionic acid ethyl ester hydrochloride

To a solution of the brown viscous oil (8.65 g) obtained in Example 1 in ethanol (69 mL) was added dropwise at room temperature a 4 mol/L hydrogen chloride-ethyl acetate solution (5.1 mL, 1 equivalent reduced to quantitative purity). After the solution was stirred for 15 minutes at room temperature, it was heated and stirred under reflux for 10 minutes. After termination of heating, the solution was allowed to gradually cool to room temperature, and then ice-cooled and stirred for 30 minutes. The resulting crystals were filtered off, washed with cold ethanol (9 mL) and then dried at 50°C under reduced pressure to give 6.9 g of the aimed product (98.0%, HPLC) as pale yellow crystals.
m.p.: 199-200°C
HPLC retention time: 5.9 minutes [column: Crestpack C18 T-5, 200 mm; solvent: acetonitrile-0.1% aqueous phosphoric acid solution (containing 5 mmol/L of sodium 1-heptanesulfonate)(35:65), detection: UV (258nm), flow rate; 1.0 mL/min].

Example 3

3-[4-(8-Fluoro-5,11-dihydrobenz[b]oxepino[4,3-b]pyridin-11-ylidene)piperidino]propionic acid hydrochloride

To an aqueous solution (16.6 mL) of sodium hydroxide (2.5 mol/L) was added the pale yellow crystals (6.4 g: 98.0%, HPLC) obtained in Example 2, and the resulting mixture was stirred for 1 hour at an internal temperature of 60°C. The reaction mixture was acidified with 6 mol/L of hydrochloric acid to pH 5 under ice-cooling, added with 51 mL of ethyl acetate and again added dropwise with 6 mol/L of hydrochloric acid to adjust the pH to 3.8. After precipitation of crystals, the solution was adjusted to a pH in a range of from 3.3 to 3.5 and stirred for 30 minutes. The resulting crystals were filtered and washed with 10 mL of isopropanol. The crystals thus obtained were dried under reduced pressure to give 5.82 g of the aimed product (99.6%, HPLC) as colorless crystals.
m.p.: 182-184°C
HPLC retention time: 6.1 minutes [column: Crestpack C18 T-5, 200 mm; solvent: acetonitrile-0.1% aqueous phosphoric acid solution (containing 5 mmol/L of sodium 1-heptanesulfonate) (30:70), detection: UV (258nm), flow rate; 1.0 mL/min].

Example 4

3-[4-(8,-Fluoro-5,11-dihydrobenz[b]oxepino[4,3-b]pyridin-11-ylidene)piperidino]propionic acid ethyl ester hydrochloride

The pale yellow crystals (0.2 g: 98.0%, HPLC) obtained in Example 2 was added to ethanol (1.6 mL) and the reaction mixture was heated under reflux for 30 minutes and then allowed to stand until it cooled to room temperature. The resulting crystals were filtered off and washed with ethanol (0.5 mL) to give 0.18 g of the aimed product (98.8%, HPLC) as colorless crystals.
m.p.: 199-201°C
HPLC retention time: 5.9 minutes [column: Crestpack C18 T-5, 200 mm; solvent: acetonitrile-0.1% aqueous phosphoric acid solution (containing 5 mmol/L of sodium 1-heptanesulfonate) (35:65), detection: UV (258nm), flow rate; 1.0 mL/min].

Example 5

3-[4-(8-Fluoro-5,11-dihydrobenz[b]oxepino[4,3-b]pyridin-11-ylidene)piperidino]propionic acid ethyl ester p-toluenesulfonate

To a solution of 3-[4-(8-Fluoro-5,11-dihydrobenz[b]oxepino[4,3-b]pyridin-11-ylidene)piperidino]propionic acid ethyl ester (0.90 g: 89.9%, HPLC) obtained in Example 1 in ethyl acetate (7.2 mL) was added at room temperature p-toluenesulfonic acid (397 mg, 1 equivalent reduced to quantitative purity). The solution was stirred for 15 minutes at room temperature, and then heated and stirred under reflux for 10 minutes. After termination of heating, the reaction mixture was allowed to stand to gradually cool to room temperature, ice-cooled and stirred for 3 hours. The resulting crystals were filtered and washed with cold ethyl acetate (0.5 mL X 2). The resulting crystals were dried under reduced pressure at 50°C to give 1.0 g of the aimed product (94.1%, HPLC) as reddish brown crystals.
m.p.: 87-89°C
HPLC retention time: 6.2 minutes [column: Crestpack C18 T-5, 200 mm; solvent: acetonitrile-0.1% aqueous phosphoric acid solution (containing 5 mmol/L of sodium 1-heptanesulfonate)(35:65), detection: UV (258nm), flow rate; 1.0 mL/min].

Example 6

3-[4-(8-Fluoro-5,11-dihydrobenz[b]oxepino[4,3-b]pyridin-11-ylidene)piperidino]propionic acid ethyl ester methanesulfonate

To a solution of 3-[4-(8-Fluoro-5,11-dihydrobenz[b]oxepino[4,3-b]pyridin-11-ylidene)piperidino]propionic acid ethyl ester (0.79 g: 89.9%, HPLC) obtained in Example 1 in ethyl acetate (6 mL) was added at room temperature methanesulfonic acid (176.2 mg, 1 equivalent reduced to quantitative purity). The solution was stirred for 15 minutes at room temperature, and then heated and stirred under reflux for 10 minutes. After termination of heating, the reaction liquid was allowed to stand to gradually cool to room temperature, ice-cooled and stirred for 2 hours. The resulting crystals were filtered and washed with cold ethyl acetate (0.5 mL × 2). The resulting crystals were dried under reduced pressure at 50°C to give 0.83 g of the aimed product (94.0%, HPLC) as brown crystals.
m.p.: 156-166°C
HPLC retention time: 6.2 minutes [column: Crestpack C18 T-5, 200 mm; solvent: acetonitrile-0.1% aqueous phosphoric acid solution (containing 5 mmol/L of sodium 1-heptanesulfonate)(35:65), detection: UV (258nm), flow rate; 1.0 mL/min].

Example 7

3-[4-(8-Fluoro-5,11-dihydrobenz[b]oxepino[4,3-b]pyridin-11-ylidene)piperidino]propionic acid ethyl ester hydrobromide

To a solution of 3-[4-(8-Fluoro-5,11-dihydrobenz[b]oxepino[4,3-b]pyridin-11-ylidene)piperidino]propionic acid ethyl ester (0.81 g: 89.9%, HPLC) obtained in Example 1 in 2-propanol (6.5 mL) was added at room temperature an acetic acid solution (30%) saturated with hydrogen bromide (0.5 mL, 1 equivalent reduced to quantitative purity). The solution was stirred for 15 minutes at room temperature, and then heated and stirred under reflux for 10 minutes. After termination of heating, the reaction mixture was allowed to stand to gradually cool to room temperature, then ice-cooled and stirred for 1 hour. The resulting crystals were filtered off and washed with cold 2-propanol (0.4 mLX2). The crystals thus obtained were dried under reduced pressure at 50°C to give 0.60 g of the aimed product (97.9%, HPLC) as reddish brown crystals.
m.p.: 204-207°C
HPLC retention time: 6.2 minutes [column: Crestpack C18 T-5, 200 mm; solvent: acetonitrile-0.1% aqueous phosphoric acid solution (containing 5 mmol/L of sodium 1-heptanesulfonate)(35:65), detection: UV (258nm), flow rate; 1.0 mL/min].

Example 8

3-[4-(8-Fluoro-5,11-dihydrobenz[b]oxepino[4,3-b]pyridin-11-ylidene)piperidino]propionic acid ethyl ester

To a suspension of zinc (8.9 g) in anhydrous tetrahydrofuran (90 mL) was added dropwise titanium tetrachloride (4.8 mL) under ice-cooling in an argon atmosphere. After the reaction mixture was stirred under reflux for 2 hours, to the boiling mixture was promptly added a solution of 8-fluoro-11-oxo-5,11-dihydrobenz[b]oxepino[4,3-b]pyridin (5.0 g) and 3-(4-oxopiperidin-1-yl)-propionic acid ethyl ester (4.35 g) in anhydrous tetrahydrofuran (68 mL) under reflux. After heating under reflux for 30 minutes, the reaction mixture was cooled to room temperature, added with ice-water, and concentrated under reduced pressure to distill away THF. After addition of toluene (200 mL) and celite (10 g), the reaction liquid was made alkaline by adding $K_2CO_3$, and filtered through celite. Celite (15 g) was added to the filtrate, and then the filtrate was further filtered. An organic layer was separated from the filtrate, an aqueous layer was extracted with toluene (100 mL), and the organic layer was combined and washed with brine (40 mL), and dried over anhydrous $MgSO_4$.

After distilling away the solvent under reduced pressure, 4.2 g of the aimed product (79.3%, HPLC) was obtained as a brown viscous oil.
HPLC retention time: 5.9 minutes [column: Crestpack C18 T-5, 200mm; solvent: MeCN-0.1% aqueous $H_3PO_4$ solution (containing 5 mM of sodium 1-heptanesulfonate)(35:65), detection: UV (258 nm), flow rate; 1.0 mL/min]]

Example 9

3-[4-(8-Fluoro-5,11-dihydrobenz[b]oxepino[4,3-b]pyridin-11-ylidene)piperidino]propionic acid ethyl ester hydrochloride

To a solution of the brown viscous oil (1.0 g) obtained in Example 8 in ethanol (8 mL) was added dropwise at room temperature a 4M hydrogen chloride ethyl acetate solution (0.4 mL, 1 equivalent reduced to quantitative purity). The solution was stirred for 15 minutes at room temperature, and then heated and stirred under reflux for 10 minutes. After termination of heating, the reaction liquid was allowed to gradually cool to room temperature, then ice-cooled and stirred for 30 minutes. The resulting crystals were filtered off, washed with cold ethanol (1 mL), and dried under reduced pressure at 50°C to give 0.54 g of the aimed product (92.8%, HPLC; quantitative purity[1)]: 92.6%) as pale yellow crystals.
Yield: 49.5%, substantial yield[2)] : 72.2%

Incidentally, 1) quantitative purity and 2) substantial yield have the following meanings:

1) Quantitative purity; Using a pure ethyl ester as a standard sample and confirming that the amount introduced to HPLC is proportional to the peak area of UV absorption, absolute quantitative determination is effected by using the ratio of the peak area of a test sample to that of the standard sample to give a quantitative purity. (Calculation example: a pure standard sample of an ethyl ester and a test sample having the same concentration are introduced in the same amount.

$$\text{quantitative purity} = (\text{peak area of the test sample}/\text{peak area of the standard sample}) \times 100$$

Quantitative purity of a hydrochloride salt; a standard sample and a test sample having the same concentration are introduced in the same amount.

$$\text{quantitative purity} = (\text{peak area of the test sample} \times a/\text{peak area of the standard sample}) \times 100$$

a=molecular weight of the hydrochloride salt [432.92]/molecular weight [396.45]=1.092)
2) Substantial yield: a value which correctly reflects the amount of an aimed product obtained according to the present reaction by utilizing the quantitative purity. substantial yield=quantitative purity after purification × yield/quantitative purity before purification
m.p.: 199-200°C
HPLC retention time: 5.9 minutes [column: Crestpack C18 T-5, 200 mm; solvent: MeCN-0.1% aqueous phosphoric acid solution (containing 5 mmol/L of sodium 1-heptanesulfonate) (35:65), detection: UV (258nm), flow rate; 1.0 mL/min].

Example 10

3-[4-(8-Fluoro-5,11-dihydrobenz[b]oxepino[4,3-b]pyridin-11-ylidene)piperidino]propionic acid ethyl ester hydrochloride

The pale yellow crystals (0.40 g; 92.8%, HPLC) obtained in Example 9 were added to ethanol (3.2 mL). After heating under reflux for 30 minutes, the reaction mixture was allowed to stand until it is cooled to room temperature. The resulting crystals were filtered and washed with ethanol (1 mL) to give 0.37 g of the aimed product (96.0%, HPLC;

quantitative purity[1)]: 100%, remaining metals: Ti;<25ppm, Zn;<2.5ppm). Yield: 92.5%, substantial yield[2)] : quantitative
1), 2): quantitative purity and substantial yield are as mentioned above.
m.p.: 199-201°C
HPLC retention time: 5.9 minutes [column: Crestpack C18 T-5, 200 mm; solvent: MeCN-0.1% aqueous $H_3PO_4$ solution (containing 5 mmol/L of sodium 1-heptanesulfonate)(35:65), detection: UV (258nm), flow rate; 1.0 mL/min].

Reference example 1

Purification of 3-[4-(8-fluoro-5,11-dihydrobenz[b]oxepino[4,3-b]pyridin-11-ylidene)piperidino]propionic acid ethyl ester

The brown viscous oil (1.0 g, 79.3%, HPLC) obtained in Example 8 was purified by column chromatography on NH silica gel (5.1 g) (chloroform: hexane=2:1) to give 928 mg of a pale brown oil (80.0%, HPLC; quantitative purity[1)]: 61.7%, remaining metals: Ti; 180ppm, Zn; 2.6ppm). Yield: 89.9%, substantial yield[2)]: 87.1%
1), 2): quantitative purity and substantial yield are as mentioned above.
HPLC retention time: 5.98 minutes [column: Crestpack C18 T-5, 200 mm; solvent: MeCN-0.1% aqueous $H_3PO_4$ solution (containing 5 mM of sodium 1-heptanesulfonate)(35:65), detection: UV (258nm), flow rate; 1.0 mL/min].

Industrial Applicability

By using as an intermediate an acid addition salt of a 3-[4-(8-fluoro-5,11-dihydrobenz[b]oxepino[4,3-b]pyridin-11-ylidene)piperidino]propionic acid alkyl ester to produce 3-[4-(8-fluoro-5,11-dihydrobenz[b]oxepino[4,3-b]pyridin-11-ylidene)piperidino]propionic acid, the metals used in the synthetic reaction step and the organic compounds mainly by-produced during production are readily separated from a reaction mixture by a simple procedure, and the by-products are sufficiently removed without using a purification step by chromatography, whereby mass production is enabled and production efficiency is enhanced.

## Claims

1. An acid addition salt of a 3-[4-(8-fluoro-5,11-dihydrobenz[b]oxepino[4,3-b]pyridin-11-ylidene)piperidino]propionic acid alkyl ester.

2. An acid addition salt of 3-[4-(8-fluoro-5,11-dihydrobenz[b]oxepino[4,3-b]pyridin-11-ylidene)piperidino]propionic acid ethyl ester.

3. The acid addition salt according to claim 1 or 2 which is a hydrochloride, hydrobromide, methanesulfonate, or p-toluenesulfonate.

4. A process for producing an acid addition salt of a 3-[4-(8-fluoro-5,11-dihydrobenz[b]oxepino[4,3-b]pyridin-11-ylidene)piperidino]propionic acid alkyl ester which comprises reacting a 3-[4-(8-fluoro-5,11-dihydrobenz[b]oxepino[4,3-b]pyridin-11-ylidene)piperidino]propionic acid alkyl ester with an acid.

5. The process for producing an acid addition salt according to claim 4, in which the reaction is conducted with heating and then the reaction system is cooled.

6. The process for producing an acid addition salt according to claim 5, in which the heating is heating under reflux.

7. The process for producing an acid addition salt according to any one of claims 4-6, in which the 3-[4-(8-fluoro-5,11-dihydrobenz[b]oxepino[4,3-b]pyridin-11-ylidene)piperidino]propionic acid alkyl ester is prepared by reacting 8-fluoro-11-oxo-5,11-dihydrobenz[b]oxepino[4,3-b]pyridin with a 3-(4-oxo-piperidin-1-yl)-propionic acid alkyl ester in the presence of a low-valent titanium, stirring the reaction mixture by air bubbles in the presence of an organic base, and then separating the product.

8. The process for producing an acid addition salt according to any one of claims 4-7, in which the acid is dissolved in an organic solvent.

9. The process for producing an acid addition salt according to claim 8, in which the organic solvent is one or more selected from the group consisting of lower alcohols and lower fatty acid esters.

**10.** The process for producing an acid addition salt according to any one of claims 4-9, in which the acid is hydrogen chloride, hydrogen bromide, methansulfonic acid or p-toluenesulfonic acid.

**11.** A process for producing 3-[4-(8-fluoro-5,11-dihydrobenz[b]oxepino[4,3-b]pyridin-11-ylidene)piperidino]propionic acid or an acid addition salt thereof which comprises hydrolyzing an acid addition salt of a 3-[4-(8-fluoro-5,11-dihydrobenz[b]oxepino[4,3-b]pyridin-11-ylidene)piperidino]propionic acid alkyl ester.

**12.** The process according to claim 11, in which the acid addition salt of a 3-[4-(8-fluoro-5,11-dihydrobenz[b]oxepino[4,3-b]pyridin-11-ylidene)piperidino]propionic acid alkyl ester is prepared by the process according to any one of claims 4-10.

**INTERNATIONAL SEARCH REPORT**

International application No.
PCT/JP2004/005304

A. CLASSIFICATION OF SUBJECT MATTER
Int.Cl[7] C07D491/044//A61K31/553, A61P37/08

According to International Patent Classification (IPC) or to both national classification and IPC

B. FIELDS SEARCHED

Minimum documentation searched (classification system followed by classification symbols)
Int.Cl[7] C07D491/044

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-338574 A (Kabushiki Kaisha Fuji Yakuhin), 27 November, 2002 (27.11.02), Full text (Family: none) | 1-12 |
| A | JP 6-116273 A (Hokuriku Seiyaku Co., Ltd.), 26 April, 1994 (26.04.94), Full text (Family: none) | 1-12 |
| A | JP 6-192263 A (Hokuriku Seiyaku Co., Ltd.), 12 July, 1994 (12.07.94), Full text (Family: none) | 1-12 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 June, 2004 (04.06.04) | 22 June, 2004 (22.06.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/005304

C (Continuation). DOCUMENTS CONSIDERED TO BE RELEVANT

| Category* | Citation of document, with indication, where appropriate, of the relevant passages | Relevant to claim No. |
|---|---|---|
| A | JP 9-124653 A (Hokuriku Seiyaku Co., Ltd.), 13 May, 1997 (13.05.97), Full text & WO 97/9330 A1 & EP 864572 A1 & US 6075035 A | 1-12 |

Form PCT/ISA/210 (continuation of second sheet) (January 2004)